# EUROPEAN PATENT SPECIFICATION

(11) **EP 4 303 212 B1**
(45) Date of publication and mention of the grant of the patent: **01.10.2025**
(21) Application number: 22762582.9
(22) Date of filing: 03.03.2022
(51) Int. Cl.: C07D 213/82, C07C 39/11, C07C 37/84

(54) **HYDROXYTYROSOL NICOTINAMIDE EUTECTIC CRYSTAL, AND PREPARATION METHOD THEREFOR AND COMPOSITION THEREOF**
EUTEKTISCHER HYDROXYTYROSOLNICOTINAMIDKRISTALL UND HERSTELLUNGSVERFAHREN DAFÜR UND ZUSAMMENSETZUNG DAVON
CRISTAL EUTECTIQUE D'HYDROXYTYROSOL-NICOTINAMIDE, SON PROCÉDÉ DE PRÉPARATION ET COMPOSITION ASSOCIÉE

(30) Priority: 05.03.2021 CN 202110243777
(43) Date of publication of application: 10.01.2024
(73) Proprietor: Cocrystal Technology (Jiaxing) Co., Ltd, Jiaxing, Zhejiang 314100 (CN)
(72) Inventor: MEI, Xuefeng, Shanghai 201203 (CN); JIANG, Yuhang, Shanghai 201203 (CN); ZHU, Bingqing, Shanghai 201203 (CN)
(74) Representative: Grünecker Patent- und Rechtsanwälte PartG mbB
(86) International application number: PCT/CN2022/078935
(87) International publication number: WO 2022/184120

(56) References cited:
- WO-A1-2014/085613
- CN-A- 105 669 543
- CN-A- 113 004 198
- US-A1- 2012 225 840

## Description

### Technical field

The invention relates to the technical field of medicines, and in particular to a co-crystal of hydroxytyrosol and nicotinamide, a preparation method therefor and a composition thereof.

### Background Art

Hydroxytyrosol is a polyphenolic compound extracted from an olive oil, with the specific structural formula as shown in formula I:

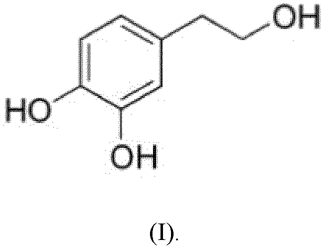

Hydroxytyrosol has a strong antioxidant capacity and a wide range of biological activities, and so far, it has been widely used in foods, health products and cosmetics. However, the hydroxytyrosol raw material itself has a low melting point (52 °C), high hygroscopicity, and is in a waxy solid form or a viscous oily state due to deliquescence under ambient temperature and humidity, so it cannot be directly used in a hard capsule or a tablet, and the raw material itself is extremely unstable to light, heat, moisture, oxygen, etc., and is easy to be oxidized and discolored during use.

By far, hydroxytyrosol had been reported to be protected by embedding, and the formed hydroxytyrosol embedding powder has a loading of 20-30%. Compared with hydroxytyrosol itself, the powder properties of the embedding powder are improved, but the embedding efficiency is not high because hydroxytyrosol is an amphiphilic compound, and the unembedded hydroxytyrosol still has problems such as instability and discoloration. What's worse, the hygroscopicity is further increased because the embedding powder contains a large amount of starch-based auxiliary materials.

In view of the above, the present invention is proposed.

### Summary of the invention

The primary object of the present invention is to provide a co-crystal of hydroxytyrosol and nicotinamide.

The second object of the present invention is to provide a method for preparing the co-crystal of hydroxytyrosol and nicotinamide.

The third object of the present invention is to provide a composition comprising the co-crystal of hydroxytyrosol and nicotinamide.

In order to accomplish the purposes of the present invention, the following technical solution is adopted.

In order to improve the stability of hydroxytyrosol products, a hydroxytyrosol co-crystal is prepared in the present invention by adding an auxiliary compound which has non-covalent bond interaction with hydroxytyrosol at the molecular level, and thus the melting point of hydroxytyrosol is capable of being increased at molecular level, and the stability of hydroxytyrosol is improved, and the application field of hydroxytyrosol is broadened.

In a first aspect, the present invention provides a co-crystal compound of hydroxytyrosol and nicotinamide, wherein the molar ratio of hydroxytyrosol to nicotinamide is 1:1, and the structural formula of nicotinamide is shown in formula II:

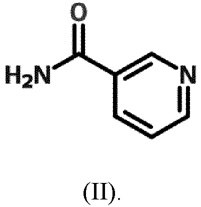

It is found through a large number of experiments in the present invention that hydroxytyrosol cannot be prepared into a stable powder by using other similar compounds such as L-proline, L-carnitine and niacin. The co-crystal prepared by using nicotinamide can solve the above-mentioned technical problems, thereby completing the present invention.

The co-crystal of hydroxytyrosol and nicotinamide has unit cell parameters of a=9.4999, b=11.8285, c=11.4439, α=90 °, β=96.628°, and δ=90°.

In some embodiments, compared with hydroxytyrosol, the co-crystal of hydroxytyrosol and nicotinamide prepared by the present invention has a significantly improved melting point. It is well known that, not all co-crystals can significantly increase the melting point, and some co-crystals may have a melting point lower than those of the two compounds alone. In some embodiments, compared with hydroxytyrosol, the hydroxytyrosol co-crystal prepared by the present invention further has significantly reduced hygroscopicity. In some embodiments, compared with hydroxytyrosol, the hydroxytyrosol co-crystal prepared by the present invention further has significantly improved chemical stability.

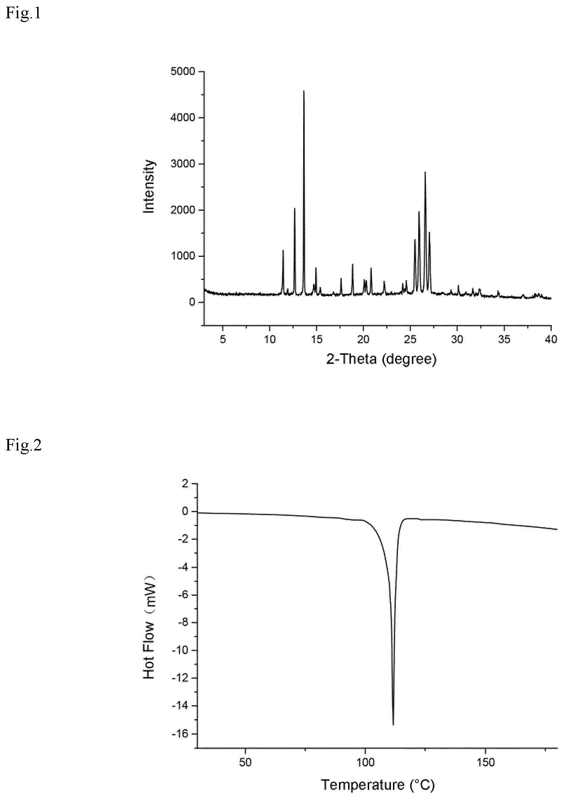

In particular, the co-crystal of hydroxytyrosol and nicotinamide has an X-ray powder diffraction pattern showing characteristic peaks at least at 2θ angles of 11.4±0.2, 13.6±0.2, 14.9±0.2, 17.6±0.2, 18.8±0.2, 20.1±0.2, 20.3±0.2, and 20.8±0.2 degrees. More particularly, the co-crystal of hydroxytyrosol and nicotinamide has an X-ray powder diffraction pattern substantially as shown in FIG. 1.

In particular, the co-crystal of hydroxytyrosol and nicotinamide has a melting point of 111 °C ± 2 °C. The co-crystal of hydroxytyrosol and nicotinamide has an onset melting temperature of 110±2 °C and a maximum peak of 111±2 °C, as determined by differential scanning calorimetry. More particularly, the co-crystal of hydroxytyrosol and nicotinamide has a differential scanning calorimetric pattern substantially as shown in FIG. 2.

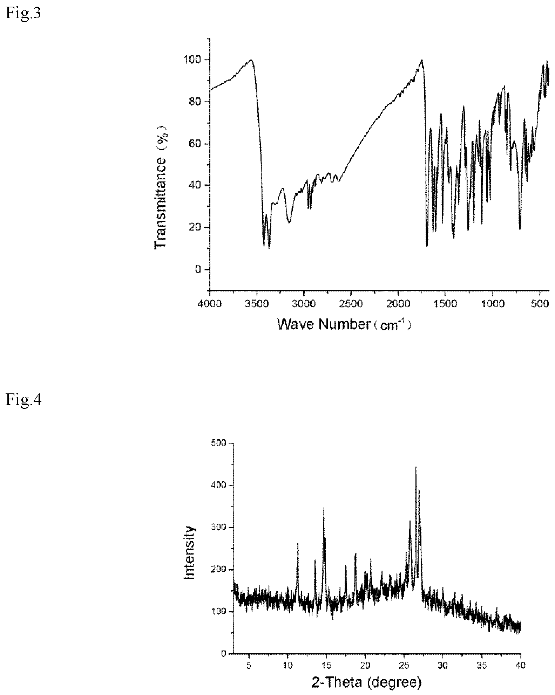

In particular, the co-crystal of hydroxytyrosol and nicotinamide has an infrared absorption spectrum showing absorption peaks at least at 3426 cm⁻¹, 3371 cm⁻¹, 3155 cm⁻¹, 1692 cm⁻¹, 1627 cm⁻¹, 1601 cm⁻¹, 1527 cm⁻¹, 1409 cm⁻¹, 1356 cm⁻¹, 1260 cm⁻¹, 1200 cm⁻¹, 1117 cm⁻¹, 1060 cm⁻¹, 1026 cm⁻¹, 929 cm⁻¹, 849 cm⁻¹, 809 cm⁻¹, 711 cm⁻¹, 654 cm⁻¹ and 635 cm⁻¹. More particularly, the co-crystal of hydroxytyrosol and nicotinamide has an infrared spectrum substantially as shown in FIG. 3.

In a second aspect, the present invention provides a method for preparing the hydroxytyrosol co-crystal, comprising a step of contacting hydroxytyrosol and nicotinamide in a molecular state and then crystallizing.

The method for contacting in the molecular state may include, but is not limited to, solution synthesis method, solid state grinding method, and the like. The solution synthesis method refers to the synthesis of co-crystal in solution, including slow evaporation, cooling crystallization, suspension crystallization, elution crystallization, and the like. The solid state grinding method mainly includes neat grinding and solvent assisted grinding. The neat grinding method is a method of obtaining a co-crystal product by grinding a mixture of the main drug and the ligand, and the solvent-assisted grinding is a method of increasing the grinding efficiency by adding a small amount of solvent during the grinding. The methods for solid-state grinding include ball milling or high-speed shearing.

In particular, the method for preparing the hydroxytyrosol co-crystal may be one of the following methods:
Method 1:
   hydroxytyrosol and nicotinamide are recrystallized in an organic solvent to obtain the co-crystal of hydroxytyrosol and nicotinamide;
Method 2:
   hydroxytyrosol and nicotinamide are mixed in a pulverizing device to obtain the co-crystal of hydroxytyrosol and nicotinamide.

In particular, the method 1 comprises at least the steps of:
S1, dissolving hydroxytyrosol and nicotinamide in an organic solvent at a first temperature;
S2, cooling the solution obtained in S1 at a second temperature lower than the first temperature to precipitate the hydroxytyrosol co-crystal;

Specifically, in S1, the first temperature is 10 to 80 °C, preferably 30 to 50 °C.

Specifically, in S1, when the co-crystal of hydroxytyrosol and nicotinamide is prepared, the organic solvent is one or more selected from the group consisting of methanol, ethanol, n-propanol, n-butanol, isopropanol, isobutanol, isoamyl alcohol, tert-butanol, acetone, methyl ethyl ketone, acetonitrile, tetrahydrofuran, nitromethane, ethyl formate, ethyl acetate, isopropyl acetate and isobutyl acetate. Preferably, the organic solvent is selected from a mixed solvent of ethanol and isoamyl alcohol, a mixed solvent of ethanol and isobutanol, and a mixed solvent of ethanol and tert-amyl alcohol. The volume ratio of the two solvents in the mixed solvents may be 1:0.1-10, preferably 1:1-5, more preferably 1:1.

Specifically, in S2, the second temperature is -40 to 0 °C, preferably -30 to -10 °C.

Specifically, in S2, crystal seeds can be added during the crystallization to accelerate the formation of crystals.

Specifically, the steps of solid-liquid separation and drying are also included after crystallization; wherein, solid-liquid separation can be carried out by filtration, centrifugation, etc., and filtration is preferred; drying can be carried out by normal pressure drying, vacuum drying, spray drying, etc., and vacuum drying at room temperature is preferred.

Specifically, the method 2 comprises at least a step of:
mixing hydroxytyrosol and nicotinamide in a pulverizing device, wherein the molar ratio of hydroxytyrosol and nicotinamide is less than or equal to 1:1.

Specifically, the pulverizing device includes a mechanical pulverizer and a ball mill.

Specifically, the mixing temperature is 15 to 50 °C.

Specifically, the frequency of the ball mill is 30 to 50 Hz.

Specifically, the rotating speed of the mechanical pulverizer is 5000 to 30000 rpm.

**In** a third aspect, the present invention provides a composition comprising the above-mentioned co-crystal of hydroxytyrosol and nicotinamide.

In some embodiments, in addition to the co-crystal of hydroxytyrosol and nicotinamide of the present invention, the composition may comprise an excess amount of nicotinamide, or an excess amount of hydroxytyrosol, as well as an other pharmaceutically acceptable excipient. That is to say, in the raw materials of the composition, the molar ratio of hydroxytyrosol to nicotinamide is not particularly limited, as long as the above-mentioned co-crystal of hydroxytyrosol and nicotinamide can be prepared from the raw materials of the composition. For example, the molar ratio of hydroxytyrosol to nicotinamide in the composition may be 10:1 to 1:10, wherein some components exist in the form of the hydroxytyrosol co-crystal, while the other components exist in free form. Further, it is preferable that all hydroxytyrosol is formed into the co-crystal to overcome the defects of low melting point and poor stability of hydroxytyrosol.

In a fourth aspect, the preparation method of the composition of the present invention is preferably performed by placing hydroxytyrosol and nicotinamide in a pulverizing device to prepare the co-crystal to obtain the composition comprising hydroxytyrosol and nicotinamide. In the raw materials of the composition, the molar ratio of hydroxytyrosol to nicotinamide is less than 1:1, preferably, the molar ratio of hydroxytyrosol to nicotinamide is 1:1.01 to 1:10, more preferably 1:1.01 to 1:3. If there is too much nicotinamide remaining, the hygroscopicity tends to increase, which is not conducive to the stability of hydroxytyrosol.

If the other pharmaceutically acceptable auxiliary material is added, the step of mixing with the other auxiliary material is further included.

In some embodiments, the raw materials of the composition are composed of hydroxytyrosol and nicotinamide in a molar ratio of 1:1.01 to 1:3, and the mole number of free nicotinamide in the composition is 101% to 200% of the mole number of hydroxytyrosol, based on the mole number of hydroxytyrosol. When the free nicotinamide in the composition is within the above ratio range, the composition has an X-ray powder diffraction pattern showing characteristic peaks at diffraction angles represented by 2θ angles of 11.4±0.2, 13.6±0.2, 14.9±0.2, 17.6±0.2, 18.8±0.2, 20.1±0.2, 20.3±0.2 and 20.8±0.2 degrees. The composition has the characteristic peaks of the co-crystal of hydroxytyrosol and nicotinamide of the present invention, thus confirming that the co-crystal in the composition is consistent with the crystal form of the co-crystal of the present invention. In particular, the composition has an onset melting point of 100 °C ± 2 °C and a maximum peak of 101±2 °C, as determined by differential scanning calorimetry. When the free nicotinamide in the composition is within the above ratio range, the composition has an infrared absorption spectrum showing absorption peaks at least at 3426 cm⁻¹, 3371 cm⁻¹, 3155 cm⁻¹, 1692 cm⁻¹, 1627 cm⁻¹, 1601 cm⁻¹, 1527 cm⁻¹, 1409 cm⁻¹, 1356 cm⁻¹, 1260 cm⁻¹, 1200 cm⁻¹, 1117 cm⁻¹, 1060 cm⁻¹, 1026 cm⁻¹, 929 cm⁻¹, 849 cm⁻¹, 809 cm⁻¹, 711 cm⁻¹, 654 cm⁻¹ and 635 cm⁻¹.

Further preferably, the composition of the present invention can be directly prepared by a grinding method. When the co-crystal of hydroxytyrosol and nicotinamide is prepared by the grinding method, the preparation time can be shortened by adding an excess amount of nicotinamide, while ensuring that all hydroxytyrosol forms into the co-crystal and no free hydroxytyrosol exists in the composition. The composition prepared directly by the grinding method has no significant adverse effects on the melting point, hygroscopicity and stability of the composition, although it contains a certain amount of nicotinamide. In addition, compared with the solvent crystallization method, it has technical advantages of high preparation efficiency, which can increase the yield and reduce the cost, and is suitable for large-scale application.

The beneficial effects of the present invention at least include the following aspects.

Compared with hydroxytyrosol itself, the co-crystal of hydroxytyrosol and nicotinamide of the present invention has significantly increased melting point. Compared with hydroxytyrosol itself and its embedding powder, the co-crystal of hydroxytyrosol and nicotinamide of the present invention has reduced hygroscopicity, as well as significantly improved chemical stability.

Compared with the co-crystal of hydroxytyrosol and nicotinamide, the composition comprising the co-crystal of hydroxytyrosol and nicotinamide prepared by a grinding method of the present invention has the technical advantages of high yield, low cost and being suitable for large-scale production.

### Brief Description of the drawings

Fig. 1 is an X-ray powder diffraction (XRPD) pattern of the co-crystal of hydroxytyrosol and nicotinamide of the example of the present invention;
Fig. 2 is a differential scanning calorimetry (DSC) pattern of the co-crystal of hydroxytyrosol and nicotinamide of the example of the present invention;
Fig. 3 is an infrared spectrum (IR) pattern of the co-crystal of hydroxytyrosol and nicotinamide of the example of the present invention;
Fig. 4 is an X-ray powder diffraction (XRPD) pattern of the composition comprising the co-crystal of hydroxytyrosol and nicotinamide of the example of the present invention;
Fig. 5 is a differential scanning calorimetry (DSC) pattern of the composition comprising the co-crystal of hydroxytyrosol and nicotinamide of the example of the present invention;
Fig. 6 is a dynamic moisture adsorption comparison pattern of hydroxytyrosol itself, hydroxytyrosol embedding powder, co-crystal of hydroxytyrosol and nicotinamide (Example 1), composition comprising co-crystal of hydroxytyrosol and nicotinamide (Example 4), and composition comprising co-crystal of hydroxytyrosol and nicotinamide (Example 5).

### Mode of the Invention

In order to make the objects, technical solution and advantages of the present invention more clear, the present invention will be further described in detail below in conjunction with the accompanying drawings and examples. It should be understood that the specific examples described here are only used to explain the present invention, not to limit the present invention.

### Reagents and instruments

The X-ray powder diffraction patterns in the examples of the present invention were recorded on a Bruker D8 Advanced type X-ray powder diffractometer, which uses Cu-Kα irradiation (λ=1.54056Å) with a scanning range of a 2θ interval from 3° to 40° with a scanning speed of 2°/min.

Differential scanning calorimetry was performed on a TA DSC Q2000 equipment with a heating rate of 10 K/min.

Thermo Scientific Nicolet 6700 was used as a Fourier transform infrared spectrometer.
Hydroxytyrosol was purchased from Shaanxi Fuheng Biotechnology Co., Ltd., with a purity of ≥98%;
Nicotinamide was purchased from Aladin Reagent, with a purity of ≥98.5%;
Hydroxytyrosol embedding powder was purchased from Shaanxi Fuheng Biotechnology Co., Ltd., and it comprises30% of hydroxytyrosol and 70% of polymer excipients (mainly maltodextrin), and was prepared by spray drying.

### Example 1

Hydroxytyrosol (4 mmol) and nicotinamide (4 mmol) in a molar ratio of 1:1 were added to 20 ml of a mixed solvent of ethanol and isoamyl alcohol (volume ratio of 1:1), and stirred at 40 °C until the solution was clear. The resultant was cooled at -20 °C and recrystallized for 24 hours to obtain a white precipitate, which was filtered through a Buchner funnel and dried in a vacuum oven at room temperature for 1 day to obtain a co-crystal of hydroxytyrosol and nicotinamide.

The unit cell parameters are a=9.4999, b=11.8285, c=11.4439, α=90°, β=96.628°, δ=90°.

It was verified from the above experimental data that the molar ratio of hydroxytyrosol to nicotinamide in the co-crystal was 1:1.

The co-crystal was characterized by X-ray powder diffraction (XRPD), differential scanning calorimetry, and infrared spectroscopy. The results are shown in Figs. 1-3.

### Example 2

Hydroxytyrosol (4 mmol) and nicotinamide (4 mmol) in a molar ratio of 1:1 were added to 20 ml of a mixed solvent of ethanol and isobutanol (volume ratio of 1:1), and stirred at 40 °C until the solution was clear. The resultant was cooled at -20 °C and recrystallized for 24 hours to obtain a white precipitate, which was filtered through a Buchner funnel and dried in a vacuum oven at room temperature for 1 day to obtain a co-crystal of hydroxytyrosol and nicotinamide.

The co-crystal was characterized by X-ray powder diffraction (XRPD), differential scanning calorimetry, and infrared spectroscopy. The results are shown in Figs. 1-3.

### Example 3

3.1 g of hydroxytyrosol and 2.4 g of nicotinamide (molar ratio of 1:1) were weighed and put in a ball mill jar (Shanghai Jingxin Tissuelyser-II sample rapid grinder). An appropriate amount of grinding balls was added thereto, and ball milling was performed at room temperature at a frequency of 40 Hz for 2 hours to obtain a co-crystal of hydroxytyrosol and nicotinamide.

The co-crystal was characterized by X-ray powder diffraction (XRPD), differential scanning calorimetry, and infrared spectroscopy. The results are shown in Figs. 1-3. It shows that the crystal form of the obtained co-crystal is the same as that of Example 1.

### Example 4

3.1 g of hydroxytyrosol and 4.8 g of nicotinamide (molar ratio of 1:2) were weighed and put in a ball mill jar (Shanghai Jingxin Tissuelyser-II sample rapid grinder). An appropriate amount of grinding balls was added thereto, and ball milling was performed at room temperature at a frequency of 40 Hz for 2 hours to obtain a composition comprising the co-crystal of hydroxytyrosol and nicotinamide.

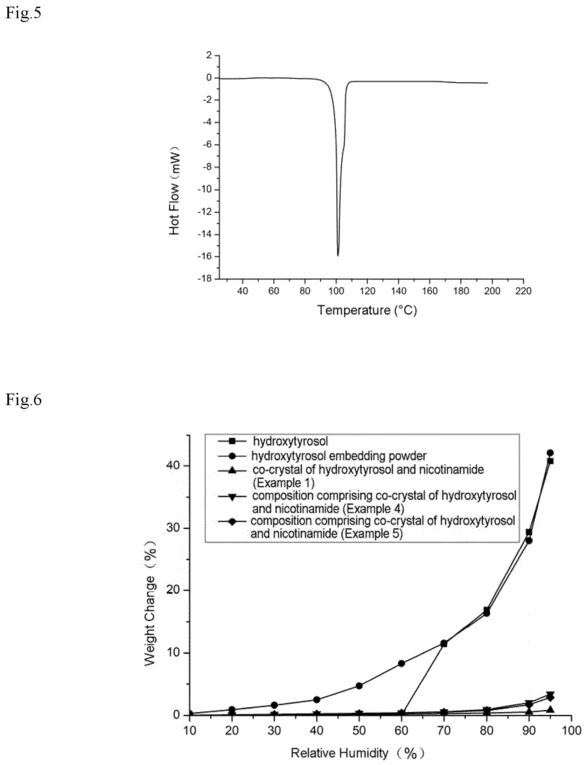

The composition was characterized by X-ray powder diffraction (XRPD), differential scanning calorimetry, and infrared spectroscopy. The results are shown in Figs. 4 and 5.

Fig. 4 shows the characteristic peaks at 11.4±0.2°, 13.6±0.2°, 14.9±0.2°, 17.6±0.2°, 18.8±0.2°, 20.1±0.2°, 20.3±0.2° and 20.8±0.2° for the co-crystal of hydroxytyrosol and nicotinamide and the characteristic peaks at 25.3±0.2° and 27.3±0.2° for the free nicotinamide crystal.

Fig. 5 shows that a eutectic mixture was formed from the hydroxytyrosol and nicotinamide co-crystal and free nicotinamide, and there was a eutectic endothermic peak at about 100 °C, without a melting peak of free nicotinamide.

It was shown from the above results that the composition is a mixture of the co-crystal of hydroxytyrosol and nicotinamide with the nicotinamide, and the crystal form was consistent with that of the co-crystal prepared in Example 1. The molar ratio of hydroxytyrosol to nicotinamide in the cocrystal was 1:1.

### Example 5

1.6 g of hydroxytyrosol and 3.8 g of nicotinamide (molar ratio is 1:3) were weighed and put in a ball mill jar (Shanghai Jingxin Tissuelyser-II sample rapid grinder). An appropriate amount of grinding balls was added thereto, and ball milling was performed at room temperature at a frequency of 40 Hz for 2 hours to obtain a composition comprising the co-crystal of hydroxytyrosol and nicotinamide, wherein the molar ratio of hydroxytyrosol to nicotinamide in the co-crystal was 1:1.

The composition was characterized by X-ray powder diffraction (XRPD), and the positions of its characteristic peaks were basically the same as those in Fig. 4, illustrating that the crystal form of the co-crystal obtained was the same as that of Example 1, and the molar ratio of hydroxytyrosol to nicotinamide in the co-crystal was 1:1. Through differential scanning calorimetry analysis, the position of its absorption peak was basically consistent with that in Fig. 5.

### Example 6

The hygroscopicity of hydroxytyrosol, hydroxytyrosol embedding powder, co-crystal of hydroxytyrosol and nicotinamide obtained in Example 1, and the composition obtained in Example 4 was compared.

About 5 mg of powder sample was weighed and put in a dynamic moisture sorption instrument (DVS). The relative humidity range was set at from 0 to 95%, and the temperature was set at 25°C. The weight change of the samples under different environmental humidity conditions were recorded and used to compare the hygroscopicity of the material. The results are shown in Fig. 6.

It can be seen from Fig. 6 that at 80% relative humidity, the hygroscopicity of the hydroxytyrosol and the embedding powder was as high as 16.9% and 16.3%, while the hydroxytyrosol co-crystal and the composition provided by the present invention were basically non-hygroscopic. The hygroscopicity of the composition (0.94% and 0.79% at 80% RH) was slightly increased compared to that of the co-crystal (0.39% at 80% RH).

It can be seen that, compared with hydroxytyrosol and its embedding powder, the hygroscopicities of the hydroxytyrosol co-crystal and the composition of the present invention were significantly lowered.

### Example 7:

The chemical stabilities of hydroxytyrosol, hydroxytyrosol embedding powder, co-crystal of hydroxytyrosol and nicotinamide obtained in Example 1, and the compositions obtained in Example 4 and Example 5 at 40°C/75%RH were compared.

An appropriate amount of powder sample was put in an accelerated stability chamber at 40°C/75%RH with the packaging condition of double-layer polyethylene bags, and the sample was taken at 0 d, 14 d, and 30 d. The content of hydroxytyrosol was determined by high performance liquid chromatography (the content was calculated by external standard method).

The conditions for the liquid chromatography were as follows:
Mobile phase: phase A: 0.1% aqueous trifluoroacetic acid solution, phase B: acetonitrile; flow rate: 1 mL/min; detection wavelength: 280 nm; column: C18 Plus 4.6×150 mm×5 µm;
Gradient:
   0 - 10 min-A:B (95:5);
   10.1 - 14 min-A:B (50:50);
   14.0 -17 min-A:B (95:5).

The experimental results are shown in Table 1.

**Table 1**

| | Day 0 | Day 14 | Day 30 |
|---|---|---|---|
| Hydroxytyrosol itself | 100.0% | 98.8% | 92.3% |
| Hydroxytyrosol embedding powder | 100.0% | 71.0% | 57.2% |
| Co-crystal of hydroxytyrosol and nicotinamide (Example 1) | 100.0% | 99.7% | 100.6% |
| Composition comprising the co-crystal of hydroxytyrosol and nicotinamide (Example 4) | 100.0% | 97.4% | 96.8% |
| Composition comprising the co-crystal of hydroxytyrosol and nicotinamide (Example 5) | 100.0% | 99.5% | 97.3% |

It can be seen from Table 1 that the initial content of hydroxytyrosol in the samples was set as 100%. After 30 days, the content of hydroxytyrosol was 92.3% of the initial content for hydroxytyrosol itself, and only 57.2% of the initial content for the embedding powder, while in the co-crystal of hydroxytyrosol and nicotinamide provided by the present invention, the content of hydroxytyrosol still remained at 99% or more. The content of hydroxytyrosol was slightly decreased in the composition comprising the co-crystal of hydroxytyrosol and nicotinamide.

It can be seen that, compared with hydroxytyrosol itself and hydroxytyrosol embedding powder, the co-crystal of hydroxytyrosol and nicotinamide and the composition of the present invention have better chemical stability.

### Comparative Example 1

Hydroxytyrosol (4 mmol) and L-proline (4 mmol) were added at a molar ratio of 1:1 to 20 ml of a mixed solvent, which was methanol, ethanol, methanol and n-propanol at a volume ratio of 1:1, methanol and n-butanol at a volume ratio of 1:1, methanol and isobutanol at a volume ratio of 1:1, methanol and isoamyl alcohol at a volume ratio of 1:1, ethanol and n-propanol at a volume ratio of 1:1, ethanol and n-butanol at a volume ratio of 1:1, ethanol and isoamyl alcohol at a volume ratio of 1:1, or ethanol and isobutanol at a volume ratio of 1:1. The mixture was stirred at 40°C until the solution was clear. The resultant was cooled at -20 °C and recrystallized for 24 hours to precipitate L-proline as a white powder, and no powder preparation of hydroxytyrosol was obtained.

It can be seen from Comparative Example 1 that a co-crystal cannot be prepared in various solvents by using L-proline.

### Comparative Example 2

Hydroxytyrosol (4 mmol) and L-carnitine (4 mmol) were added at a molar ratio of 1:1 to 20 ml of a mixed solvent, which was methanol, ethanol, methanol and n-propanol at a volume ratio of 1:1, methanol and n-butanol at a volume ratio of 1:1, methanol and isobutanol at a volume ratio of 1:1, methanol and isoamyl alcohol at a volume ratio of 1:1, ethanol and n-propanol at a volume ratio of 1:1, ethanol and n-butanol at a volume ratio of 1:1, ethanol and isoamyl alcohol at a volume ratio of 1:1, or ethanol and isobutanol at a volume ratio of 1:1. The mixture was stirred at 40°C until the solution was clear. The resultant was cooled at -20 °C and recrystallized for 24 hours to precipitate L-carnitine as a white powder, and no powder preparation of hydroxytyrosol was obtained.

It can be seen from Comparative Example 2 that a co-crystal cannot be prepared in various solvents by using L-carnitine.

### Comparative Example 3

Hydroxytyrosol (4 mmol) and nicotinic acid (4 mmol) were added at a molar ratio of 1:1 to 20 ml of a mixed solvent, which was methanol, ethanol, methanol and n-propanol at a volume ratio of 1:1, methanol and n-butanol at a volume ratio of 1:1, methanol and isobutanol at a volume ratio of 1:1, methanol and isoamyl alcohol at a volume ratio of 1:1, ethanol and n-propanol at a volume ratio of 1:1, ethanol and n-butanol at a volume ratio of 1:1, ethanol and isoamyl alcohol at a volume ratio of 1:1, or ethanol and isobutanol at a volume ratio of 1:1. The mixture was stirred at 40°C until the solution was clear. The resultant was cooled at -20 °C and recrystallized for 24 hours to precipitate nicotinic acid ligand, and no powder preparation of hydroxytyrosol was obtained.

It can be seen from Comparative Example 3 that a co-crystal cannot be prepared in various solvents by using nicotinic acid.

Comparing the results of the Examples and Comparative Examples, it can be found that hydroxytyrosol can only form a co-crystal with a specific compound, such as nicotinamide, while hydroxytyrosol cannot form a co-crystal with the structural analogs, such as L-proline, L-carnitine, nicotinic acid, and the like.

Although the present application is disclosed as above with preferred embodiments, they are not used to limit the claims. Any person skilled in the art can make some possible changes and modifications without departing from the concept of the present application. Therefore, the protection scope of the present application should be defined by the claims of the present application.

## Claims

1. A co-crystal of hydroxytyrosol and nicotinamide, wherein the co-crystal of hydroxytyrosol and nicotinamide has a molar ratio of hydroxytyrosol to nicotinamide of 1:1.

2. The co-crystal of hydroxytyrosol and nicotinamide according to claim 1, wherein the co-crystal of hydroxytyrosol and nicotinamide has unit cell parameters of a=9.4999, b=11.8285, c=11.4439, α=90 °, β=96.628°, and δ=90°.

3. The co-crystal of hydroxytyrosol and nicotinamide according to claim 1, wherein the co-crystal of hydroxytyrosol and nicotinamide has an X-ray powder diffraction pattern showing characteristic peaks at least at 2θ angles of 11.4±0.2, 13.6±0.2, 14.9±0.2, 17.6±0.2, 18.8±0.2, 20.1±0.2, 20.3±0.2, and 20.8±0.2 degrees; and/or
the co-crystal of hydroxytyrosol and nicotinamide has an X-ray powder diffraction pattern substantially as shown in FIG. 1.

4. The co-crystal of hydroxytyrosol and nicotinamide according to claim 1, wherein the co-crystal of hydroxytyrosol and nicotinamide has a melting point of 111 °C ± 2 °C; and/or
the co-crystal of hydroxytyrosol and nicotinamide has an onset melting temperature of 110±2 °C and a maximum peak of 111±2 °C, as determined by differential scanning calorimetry.

5. The co-crystal of hydroxytyrosol and nicotinamide according to claim 1, wherein the co-crystal of hydroxytyrosol and nicotinamide has an infrared absorption spectrum showing absorption peaks at least at 3426 cm⁻¹, 3371 cm⁻¹, 3155 cm⁻¹, 1692 cm⁻¹, 1627 cm⁻¹, 1601 cm⁻¹, 1527 cm⁻¹, 1409 cm⁻¹, 1356 cm⁻¹, 1260 cm⁻¹, 1200 cm⁻¹, 1117 cm⁻¹, 1060 cm⁻¹, 1026 cm⁻¹, 929 cm⁻¹, 849 cm⁻¹, 809 cm⁻¹, 711 cm⁻¹, 654 cm⁻¹ and 635 cm⁻¹;
preferably, the co-crystal of hydroxytyrosol and nicotinamide has an infrared spectrum substantially as shown in FIG. 3.

6. A method for preparing the co-crystal of hydroxytyrosol and nicotinamide according to any one of claims 1 to 5, wherein the method comprises at least the steps of:
S1, dissolving hydroxytyrosol and nicotinamide in an organic solvent, and stirring at a first temperature until completely dissolved;
S2, cooling the solution obtained in S1 and crystallizing at a second temperature lower than the first temperature;
S3, separating and drying the solid to obtain the co-crystal of hydroxytyrosol and nicotinamide;
wherein the first temperature is 10 to 80 °C, preferably 30 to 50 °C; and
wherein the second temperature is -40 to 0 °C, preferably -30 to -10 °C.

7. The method according to claim 6, wherein the organic solvent is one or more selected from the group consisting of methanol, ethanol, n-propanol, n-butanol, isopropanol, isobutanol, isoamyl alcohol, tert-butanol, acetone, methyl ethyl ketone, acetonitrile, tetrahydrofuran, nitromethane, ethyl formate, ethyl acetate, isopropyl acetate and isobutyl acetate.

8. A method for preparing the co-crystal of hydroxytyrosol and nicotinamide according to any one of claims 1 to 5, wherein the method comprises at least a step of:
mixing and grinding hydroxytyrosol and nicotinamide in a pulverizing device, wherein the molar ratio of hydroxytyrosol to nicotinamide is less than or equal to 1:1;
the pulverizing device includes a mechanical pulverizer and a ball mill;
the mixing temperature is 15 to 50 °C; and
the frequency of the ball mill is 30 to 50 Hz.

9. A composition comprising the co-crystal of hydroxytyrosol and nicotinamide according to any one of claims 1 to 5, wherein the composition further comprises nicotinamide and/or a pharmaceutically acceptable excipient;
preferably, when the raw material of the composition is composed of hydroxytyrosol and nicotinamide with a molar ratio of 1:1.01 to 1:3, the composition has an X-ray powder diffraction pattern showing characteristic peaks at 2θ angles of 11.4±0.2, 13.6±0.2, 14.9±0.2, 17.6±0.2, 18.8±0.2, 20.1±0.2, 20.3±0.2 and 20.8±0.2 degrees.

10. A method for preparing the composition of claim 9, wherein the method is a solid state grinding method;
preferably, the method comprises at least a step of:
mixing and grinding hydroxytyrosol and nicotinamide in a pulverizing device, wherein the molar ratio of hydroxytyrosol to nicotinamide is less than 1:1, preferably 1:1.01 to 1:5, more preferably 1:1.01 to 1:3.

## Patentansprüche

1. Co-Kristall aus Hydroxytyrosol und Nicotinamid, wobei der Co-Kristall aus Hydroxytyrosol und Nicotinamid ein Molverhältnis von Hydroxytyrosol zu Nicotinamid von 1:1 aufweist.

2. Co-Kristall aus Hydroxytyrosol und Nicotinamid nach Anspruch 1, wobei der Co-Kristall aus Hydroxytyrosol und Nicotinamid Einheitszellenparameter von a = 9,4999, b = 11,8285, c = 11,4439, α = 90 °, β = 96,628° und δ = 90° aufweist.

3. Co-Kristall aus Hydroxytyrosol und Nicotinamid nach Anspruch 1, wobei der Co-Kristall aus Hydroxytyrosol und Nicotinamid ein Röntgenpulverbeugungsdiagramm aufweist, das charakteristische Peaks bei mindestens 2 θ Winkeln von 11,4 ± 0,2, 13,6 ± 0,2, 14,9 ± 0,2, 17,6 ± 0,2, 18,8 ± 0,2, 20,1 ± 0,2, 20,3 ± 0,2 und 20,8 ± 0,2 Grad zeigt; und/oder
der Co-Kristall aus Hydroxytyrosol und Nicotinamid ein Röntgenpulverbeugungsdiagramm aufweist, das im Wesentlichen der Darstellung in FIG. 1 gleicht.

4. Co-Kristall aus Hydroxytyrosol und Nicotinamid nach Anspruch 1, wobei der Co-Kristall aus Hydroxytyrosol und Nicotinamid einen Schmelzpunkt von 111 °C ± 2 °C aufweist; und/oder
der Co-Kristall aus Hydroxytyrosol und Nicotinamid eine Anfangsschmelztemperatur von 110 ± 2 °C und einen maximalen Peak von 111 ± 2 °C aufweist, wie durch Differentialscanningkalorimetrie bestimmt.

5. Co-Kristall aus Hydroxytyrosol und Nicotinamid nach Anspruch 1, wobei der Co-Kristall aus Hydroxytyrosol und Nicotinamid ein Infrarot-Absorptionsspektrum aufweist, das Absorptionspeaks mindestens bei 3426 cm⁻¹, 3371 cm⁻¹, 3155 cm⁻¹, 1692 cm⁻¹, 1627 cm⁻¹, 1601 cm⁻¹, 1527 cm⁻¹, 1409 cm⁻¹, 1356 cm⁻¹, 1260 cm⁻¹, 1200 cm⁻¹, 1117 cm⁻¹, 1060 cm⁻¹, 1026 cm⁻¹, 929 cm⁻¹, 849 cm⁻¹, 809 cm⁻¹, 711 cm⁻¹, 654 cm⁻¹ und 635 cm⁻¹ zeigt;
wobei der Co-Kristall aus Hydroxytyrosol und Nicotinamid ein Infrarotspektrum aufweist,
das im Wesentlichen der Darstellung in FIG. 3 gleicht.

6. Verfahren zum Herstellen des Co-Kristalls aus Hydroxytyrosol und Nicotinamid nach einem der Ansprüche 1 bis 5, wobei das Verfahren mindestens die folgenden Schritte umfasst:
S1, Lösen von Hydroxytyrosol und Nikotinamid in einem organischen Lösungsmittel und Rühren bei einer ersten Temperatur, bis sie vollständig gelöst sind;
S2, Abkühlen der in S1 erhaltenen Lösung und Kristallisieren bei einer zweiten Temperatur, die niedriger als die erste Temperatur ist;
S3, Separieren und Trocknen des Festkörpers, um den Co-Kristall aus Hydroxytyrosol und Nicotinamid zu erhalten;
wobei die erste Temperatur 10 bis 80 °C, vorzugsweise 30 bis 50 °C beträgt; und
wobei die zweite Temperatur -40 bis 0 °C, vorzugsweise -30 bis -10 °C beträgt.

7. Verfahren nach Anspruch 6, wobei das organische Lösungsmittel eines oder mehrere ist, das aus der Gruppe bestehend aus Methanol, Ethanol, n-Propanol, n-Butanol, Isopropanol, Isobutanol, Isoamylalkohol, tert-Butanol, Aceton, Methylethylketon, Acetonitril, Tetrahydrofuran, Nitromethan, Ethylformiat, Ethylacetat, Isopropylacetat und Isobutylacetat ausgewählt ist.

8. Verfahren zum Herstellen des Co-Kristalls aus Hydroxytyrosol und Nicotinamid nach einem der Ansprüche 1 bis 5, wobei das Verfahren mindestens einen Schritt zu Folgendem umfasst:
Mischen und Mahlen von Hydroxytyrosol und Nicotinamid in einer Pulverisierungsvorrichtung, wobei das Molverhältnis von Hydroxytyrosol zu Nicotinamid kleiner oder gleich 1:1 ist;
wobei die Pulverisierungsvorrichtung einen mechanischen Zerkleinerer und eine Kugelmühle beinhaltet;
die Mischtemperatur 15 bis 50 °C beträgt; und
die Frequenz der Kugelmühle 30 bis 50 Hz beträgt.

9. Zusammensetzung, umfassend den Co-Kristall aus Hydroxytyrosol und Nicotinamid nach einem der Ansprüche 1 bis 5, wobei die Zusammensetzung des Weiteren Nicotinamid und/oder einen pharmazeutisch verträglichen Hilfsstoff umfasst;
wobei, wenn das Rohmaterial der Zusammensetzung aus Hydroxytyrosol und Nikotinamid in einem Molverhältnis von 1:1,01 bis 1:3 besteht, die Zusammensetzung vorzugsweise ein Röntgenpulverbeugungsdiagramm aufweist, das charakteristische Peaks bei 2 θ Winkeln von 11,4 ± 0,2, 13,6 ± 0,2, 14,9 ± 0,2, 17,6 ± 0,2, 18,8 ± 0,2, 20,1 ± 0,2, 20,3 ± 0,2 und 20,8 ± 0,2 Grad zeigt.

10. Verfahren zum Herstellen der Zusammensetzung nach Anspruch 9, wobei das Verfahren ein Festkörpermahlverfahren ist;
wobei das Verfahren vorzugsweise mindestens einen Schritt zu Folgendem umfasst:
Mischen und Mahlen von Hydroxytyrosol und Nicotinamid in einer Pulverisierungsvorrichtung, wobei das Molverhältnis von Hydroxytyrosol zu Nicotinamid kleiner oder gleich 1:1 ist, vorzugweise 1:1,01 bis 1:5, noch bevorzugter 1:1,01 bis 1:3.

## Revendications

1. Co-cristal d'hydroxytyrosol et de nicotinamide, le co-cristal d'hydroxytyrosol et de nicotinamide ayant un rapport molaire de l'hydroxytyrosol au nicotinamide de 1:1.

2. Co-cristal d'hydroxytyrosol et de nicotinamide selon la revendication 1, le co-cristal d'hydroxytyrosol et de nicotinamide ayant comme paramètres de maille unitaire a = 9,4999, b = 11,8285, c = 11,4439, α = 90°, β = 96,628° et δ = 90°.

3. Co-cristal d'hydroxytyrosol et de nicotinamide selon la revendication 1, le co-cristal d'hydroxytyrosol et de nicotinamide ayant un diffractogramme de rayons X sur poudre qui présente des pics caractéristiques au moins aux angles 2θ de 11,4 ± 0,2, 13,6 ± 0,2, 14,9 ± 0,2, 17,6 ± 0,2, 18,8 ± 0,2, 20,1 ± 0,2, 20,3 ± 0,2 et 20,8 ± 0,2 degrés ; et/ou
le co-cristal d'hydroxytyrosol et de nicotinamide ayant un diffractogramme de rayons X sur poudre sensiblement comme présenté à la FIG. 1.

4. Co-cristal d'hydroxytyrosol et de nicotinamide selon la revendication 1, le co-cristal d'hydroxytyrosol et de nicotinamide ayant un point de fusion de 111 °C ± 2 °C ; et/ou
le co-cristal d'hydroxytyrosol et de nicotinamide ayant une température de début de fusion de 110 °C ± 2 °C et un pic maximal de 111 °C ± 2 °C, ainsi que déterminé par analyse calorimétrique différentielle à compensation de puissance.

5. Co-cristal d'hydroxytyrosol et de nicotinamide selon la revendication 1, le co-cristal d'hydroxytyrosol et de nicotinamide ayant un spectre d'absorption infrarouge qui présente des pics d'absorption au moins à 3426 cm⁻¹, 3371 cm⁻¹, 3155 cm⁻¹, 1692 cm⁻¹, 1627 cm⁻¹, 1601 cm⁻¹, 1527 cm⁻¹, 1409 cm⁻¹, 1356 cm⁻¹, 1260 cm⁻¹, 1200 cm⁻¹, 1117 cm⁻¹, 1060 cm⁻¹, 1026 cm⁻¹, 929 cm⁻¹, 849 cm⁻¹, 809 cm⁻¹, 711 cm⁻¹, 654 cm⁻¹, et 635 cm⁻¹ ;
le co-cristal d'hydroxytyrosol et de nicotinamide ayant de préférence un spectre d'absorption infrarouge sensiblement comme présenté à la FIG. 3.

6. Procédé de préparation du co-cristal d'hydroxytyrosol et de nicotinamide selon l'une quelconque des revendications 1 à 5, le procédé comprenant au moins les étapes consistant à :
S1, dissoudre de l'hydroxytyrosol et du nicotinamide dans un solvant organique, et agiter à une première température jusqu'à dissolution complète ;
S2, refroidir la solution obtenue à l'étape S1 et laisser cristalliser à une deuxième température inférieure à la première température ;
S3, séparer et sécher le solide afin d'obtenir le co-cristal d'hydroxytyrosol et de nicotinamide ;
la première température allant de 10 à 80 °C, de préférence de 30 à 50 °C ; et
la deuxième température allant de -40 à 0 °C, de préférence de -30 à -10 °C.

7. Procédé selon la revendication 6, le solvant organique étant un ou plusieurs solvants choisis dans le groupe constitué par le méthanol, l'éthanol, le n-propanol, le n-butanol, l'isopropanol, l'isobutanol, l'alcool isoamylique, le tert-butanol, l'acétone, la méthyl-éthyl-cétone, l'acétonitrile, le tétrahydrofurane, le nitromé-thane, le formiate d'éthyle, l'acétate d'éthyle, l'acétate d'isopropyle et l'acétate d'isobutyle.

8. Procédé de préparation du co-cristal d'hydroxytyrosol et de nicotinamide selon l'une quelconque des revendications 1 à 5, le procédé comprenant au moins une étape consistant à :
mélanger et broyer l'hydroxytyrosol et le nicotinamide dans un dispositif de pulvérisation, le rapport molaire de l'hydroxytyrosol au nicotinamide étant inférieur ou égal à 1:1 ;
le dispositif de pulvérisation comportant un pulvérisateur mécanique et un broyeur à boulets ;
la température de mélange allant de 15 à 50 °C ; et
la fréquence du broyeur à boulets allant de 30 à 50 Hz.

9. Composition comprenant le co-cristal d'hydroxytyrosol et de nicotinamide selon l'une quelconque des revendications 1 à 5, la composition comprenant en outre du nicotinamide et /ou un excipient pharmaceutiquement acceptable ;
où de préférence, lorsque la matière première de la composition est composée d'hydroxytyrosol et de nicotinamide dans un rapport molaire de 1:1,01 à 1:3, la composition a un diffractogramme de rayons X sur poudre qui présente des pics caractéristiques aux angles 2θ de 11,4 ± 0,2, 13,6 ± 0,2, 14,9 ± 0,2, 17,6 ± 0,2, 18,8 ± 0,2, 20,1 ± 0,2, 20,3 ± 0,2 et 20,8 ± 0,2 degrés.

10. Procédé de préparation de la composition selon la revendication 9, le procédé étant un procédé de broyage à l'état solide ;
le procédé comprenant de préférence au moins une étape consistant à :
mélanger et broyer de l'hydroxytyrosol et du nicotinamide dans un dispositif de pulvérisation, le rapport molaire de l'hydroxytyrosol au nicotinamide étant inférieur à 1:1, de préférence de 1:1,01 à 1:5, plus préférablement de 1:1,01 à 1:3.
